# EUROPÄISCHE PATENTANMELDUNG

(11) **EP 3 312 610 A1**
(43) Veröffentlichungstag der Anmeldung: **25.04.2018**
(21) Anmeldenummer: 16194496.2
(22) Anmeldetag: 19.10.2016
(51) Int. Cl.: G01N 33/569

(54) **VERFAHREN ZUR IMMUNOLOGISCHEN DIAGNOSE EINER PROBE MIT EINER POTENTIELLEN INFEKTION EINES ARBOVIRUS UND HIERFÜR GEEIGNETE TESTKITS**

(71) Anmelder: Mikrogen GmbH, 82061 Neuried (DE)
(72) Erfinder: Soutschek, Erwin, 82335 Berg (DE); Böcher, Oliver, 82061 Neuried (DE); Nölting, Christina, 80686 München (DE)
(74) Vertreter: Keller, Günter

(57) **Zusammenfassung**

Die vorliegende Erfindung betrifft ein Verfahren zur immunologischen Diagnose einer Probe eines Patienten mit einer potentiellen Infektion eines Arbovirus, worin
a) eine Probe mit mehreren voneinander getrennt auf einer Festphase aufgebrachten Antigenen in Kontakt gebracht wird, wobei wenigstens folgende Antigene eingesetzt werden:
aa) das "non-structural protein 1" eines ersten Arbovirus oder ein immunologisch reaktives Teil hiervon mit wenigstens 8 Aminosäuren,
bb) ein "envelope protein" eines ersten Arbovirus oder ein immunologisch reaktives Teil hiervon mit wenigstens 8 Aminosäuren,
cc) ein "envelope protein" eines zweiten Arbovirus oder ein immunologisch reaktives Teil hiervon mit wenigstens 8 Aminosäuren,
dd) das "non-structural protein 1" eines dritten Arbovirus oder ein immunologisch reaktives Teil hiervon mit wenigstens 8 Aminosäuren und
ee) ein "envelope protein" eines dritten Arbovirus oder ein immunologisch reaktives Teil hiervon mit wenigstens 8 Aminosäuren;

b) Waschen der Festphase, um unspezifische Bindungen abzutrennen,
c) Umwandeln des auf der Festphase gebildeten Immunkomplexes in ein Signal und
d) Auswerten des Testverfahrens durch Vergleich der relativen Signalstärken.

## Beschreibung

Gegenstand der vorliegenden Erfindung ist ein Verfahren zur immunologischen Diagnose einer Probe eines Patienten mit einer potentiellen Infektion eines Arbovirus und hierfür geeignete Test-Kits.

Viren, die durch Insekten (vor allem Mücken) oder Spinnentiere (beispielsweise Zecken) übertragen werden, bezeichnet man als Arboviren (arthropod-borne viruses). Voraussetzung für die Übertragung von Viren durch Arthropoden ist, dass die Viren bestimmte Organe der Tiere (vor allem Stechmücken), wie etwa die Epithelzellen des Darms oder der Speicheldrüsen, infizieren können. Grundsätzlich genügt die alleinige Aufnahme von virushaltigem Blut durch Arthropoden nicht. Vielmehr müssen die Viren sowohl in Arthropoden, wie auch in Säugetierzellen einen produktiven Infektionszyklus durchlaufen können.

Durch den in den letzten Jahren äußerst stark zugenommenen Reiseverkehr und den globalen Handel sind sowohl Viren, wie auch Mücken in Gegenden verbracht worden, in denen sie ursprünglich nicht endemisch waren. Dadurch können Erkrankungen und lokale Epidemien auch an solchen geographischen Orten auftreten, an denen sie vorher nicht beobachtet wurden. Weiterhin spielt die starke Industrialisierung und die damit verbundene zunehmende Bevölkerungsdichte eine wesentliche Rolle für die Verbreitung von Epidemien.

Ein großer Teil der Bevölkerung reist in geographisch weit entfernte Gegenden und es ist daher häufig diagnostisch schwierig zu bestimmen, mit welchem Virus der Patient infiziert wurde. Darüber hinaus ist es durchaus möglich, dass Infektionen mit mehreren verschiedenen Viren vorliegen und der Patient kann schon vorher eine möglicherweise inapparent verlaufene Virusinfektion durchlaufen haben. Ein weiteres Problem sind Kreuzreaktivitäten, die aufgrund der genetischen Verwandtschaft der Viren und der Ähnlichkeiten der Antigene auftreten können. Eine weitere Komplikation der Diagnostik kann durch Impfungen (beispielsweise gegen das Gelbfieber-Virus) hervorgerufen werden. Cleton et al. (PLOS Neglected Tropical Diseases, März 2015, S. 1-17) weisen auf die Schwierigkeiten einer Differenzialdiagnostik bei Infektionen durch ein Mitglied der Familie Flaviviridae hin. In dem dort beschriebenen Test werden die NS1-Proteine für die immunologische Diagnostik verwendet.

Erfindungsgemäß bevorzugt ist eine immunologische Differenzialdiagnose zur Unterscheidung einer Infektion durch Dengue-Virus, Zika-Virus sowie Chikungunya-Virus. Weiterhin kann auch der Nachweis einer Gelbfieber-Infektion oder -Impfung gezeigt werden, sowie die differentialdiagnostische Unterscheidung.

Bei dem Dengue-Virus handelt es sich um ein Virus mit einzelsträngigem RNA-Genom in Plusstrang-Orientierung. Das Dengue-Virus gehört zu dem Genus Flavivirus der Familie Flaviviridae. Zu dieser Familie gehört auch das Gelbfieber-Virus, das West-Nil-Virus, das japanische Encephalitis-Virus, das St. Louis-Encephalititis-Virus und das Frühsommer-Menengitis-Virus, das auch im Voralpenland gehäuft auftreten kann.

Zu dem Genus Flavivirus gehört auch das Zika-Virus. Im Jahr 1947 wurde das Zika-Virus in Uganda entdeckt und es wurden nur sporadische Ausbrüche des Virus beobachtet. In den letzten Jahren jedoch ist es zu einer explosionsartigen Ausbreitung des Virus gekommen, wobei sich die klinischen Symptome von nahezu symptomfrei, über eine milde fiebrige Erkrankung bis zu schweren Fällen mit neurologischen Komplikationen erstrecken können. Mittlerweile geht man davon aus, dass das Zika-Virus auch durch sexuelle Aktivitäten übertragen werden kann. Bei Infektionen während der Schwangerschaft wurden häufig Fälle von Mikrocephalie beobachtet. Eine zuverlässige Differenzialdiagnostik ist daher insbesondere dann wichtig, wenn es sich bei der Patientin um eine schwangere Frau handelt. Weiterhin ist zu erwähnen, daß das Zika-Virus die höchste genetische Homologie zu dem Dengue-Viren besitzt, was in der Regel ein Problem bei deren Differenzierung darstellt.

Ein anderes zu den Arboviren zählendes Virus ist das Chikungunya-Virus, das zu der Familie der Togaviridae, Gattung Alphavirus, gehört. Dengue-, Zika- sowie Chikungunya-Viren werden auf den Menschen durch Stechmücken der Gattung *Aedes* übertragen und können sehr ähnliche klinische Symptome wie Fieber, Schmerzen in den Gelenken, Kopfschmerzen, Müdigkeit, Schwindel und Erbrechen hervorrufen.

Aufgabe der vorliegenden Erfindung ist es, ein Verfahren zur immunologischen Diagnose einer mutmaßlichen Infektion mit einem oder mehreren Arbovirus bereitzustellen, wobei ermittelt werden soll, mit welchen Viren der Patient / die Patientin infiziert worden ist.

In den ersten Tagen nach Auftreten der Symptome kann der Erreger mittels Nukleinsäure-Nachweis ermittelt werden. Gegen Ende der Akutphase ist dies jedoch i.d.R. nicht mehr möglich. Dann ist nach internationalen Richtlinien (WHO, CDC) ein serologischer Nachweis die Methode der Wahl.

Weiterhin hat ein immunologisches Diagnoseverfahren den Vorteil, dass die Titer von IgM bzw. IgG dazu verwendet werden können, den Verlauf der Erkrankung zu charakterisieren (akute Phase, kürzlich zurückliegend und abgelaufene/weit zurückliegende Infektion). Nach einer Infektion mit einem Arbovirus steigt zunächst der IgM-Titer stark an und fällt dann deutlich ab. Der IgG-Titer steigt leicht zeitverzögert an und verbleibt auf einem höheren Niveau als der IgM-Titer. Bei einem erneuten Kontakt mit dem Erreger steigt der IgG-Titer sehr stark an, wohingegen der IgM-Titer nicht so stark ansteigt bzw. nach spätestens 3 Monaten nach Infektion nicht mehr nachweisbar ist.

Gegenstand der vorliegenden Erfindung ist ein Verfahren zur immunologischen Diagnostik, insbesondere der Differenzialdiagnostik, einer Probe eines Patienten / einer Patientin auf eine potentielle Infektion mit einem Arbovirus.

Bei dem Verfahren wird zunächst die Probe des Patienten in Kontakt gebracht mit mehreren Antigenen, die räumlich getrennt voneinander auf einer Festphase aufgebracht wurden. Die Probe ist in bevorzugter Ausführungsform eine Antikörper enthaltende Probe, also eine Serum- und/oder Plasmaprobe. Andere Körperflüssigkeiten eines Patienten, wie beispielsweise Liquor oder Muttermilch, können in dem Verfahren ebenfalls eingesetzt werden.

Bei den Antigenen werden mehrere unterschiedliche Proteine oder Peptidfragmente mit wenigstens 8, bevorzugt 12 und ganz bevorzugt 40 Aminosäuren eingesetzt. Bei den erfindungsgemäßen Verfahren bzw. den Kits zur Durchführung dieser Verfahren werden die nachfolgend näher aufgeführten Antigene eingesetzt. Es ist möglich, die vollständigen Proteine einzusetzen, oder auch Fragmente dieser Proteine mit den immunologisch reaktiven Teilen. Da die Sequenzen der Antigene bekannt sind, können die Proteine bzw. Proteinfragmente in an sich bekannter Art und Weise rekombinant hergestellt werden. Um herauszufinden, welche Teile immunologisch reaktiv sind, werden üblicherweise diejenigen Teile exprimiert, die sich bei den Antigenen an Stellen befinden, die dem Immunsystem präsentiert werden. Welche Teile besonders geeignet sind, kann leicht dadurch ermittelt werden, dass die exponierten Fragmente rekombinant hergestellt werden und mit bekannten Patientenseren getestet werden. Diese bekannten, wohl definierten Patientenseren stammen von solchen Patienten, bei denen die Infektion mit einem bestimmten Virus über die verschiedenen Diagnosemöglichkeiten sicher nachgewiesen wurde.

Wenn Fragmente eingesetzt werden, weisen diese wenigstens 8, in der Regel wenigstens 40, bevorzugt wenigstens 60 und besonders bevorzugt wenigstens 80 Aminosäuren auf. Besonders bevorzugt sind auch solche Antigene, die dem Volllängenprotein entsprechen oder wenigstens 80 %, bevorzugt wenigstens 90 % und ganz bevorzugt wenigstens 95 % des Volllängenantigens beinhalten. In bevorzugter Ausführungsform werden entweder am N-Terminus oder am C-Terminus oder an beiden Enden Fragmente von wenigstens 10, bevorzugt wenigstens 20 Aminosäuren nicht exprimiert.

Es handelt sich hierbei bevorzugt um folgende Antigene:

### a) "non-structural protein 1" (NS1 Antigen) des Dengue-Virus

Es gibt vier Serotypen des Dengue-Virus mit den Bezeichnungen DENV-1, DENV-2, DENV-3 und DENV-4. Erfindungsgemäß wird das NS1 des Serotyps 2 (DENV-2) eingesetzt. Das NS1 ist ein hochkonserviertes Glykoprotein, das scheinbar für die Überlebensfähigkeit des Virus essentiell ist, aber es wurde diesem Antigen noch keine eindeutige biologische Aktivität zugeordnet. Eigentlich unüblich für ein virales Glykoprotein wird NS1 sowohl in Membran-assoziierter, wie auch in sekretierter Form produziert. Daher ist das NS1 Antigen während der frühen klinischen Phase der Erkrankung in hohen Konzentrationen im Serum von mit Dengue-Virus infizierten Patienten vorhanden. Die kompletten Genom-Sequenzen der Dengue-Virus-Typen 1-4 wurden von Añez et al., Genome Announcements (2016), Vol. 4, No. 1, S. 1-2 veröffentlicht.

### b) "envelope protein" des Dengue-Virus

Ein weiteres, bei dem erfindungsgemäßen Testverfahren eingesetztes Protein ist das "envelope protein" des Dengue-Virus Serotyp 2. Das envelope/Hüllprotein E stellt das Hauptantigen für die humorale Immunantwort dar. Hierin auftretende Mutationen können die Virulenz des Dengue-Virus beeinflussen.

### c) "envelope protein 1" des Chikungunya-Virus

Ein weiteres, in dem Test eingesetztes Antigen ist das "envelope protein 1" des Chikungunya-Virus oder ein immunologisch reaktives Teil hiervon mit wenigstens 40 Aminosäuren. Zwischenzeitlich sind drei phylogenetisch unterscheidbare Gruppen des Chikungunya-Virus bekannt, nämlich der asiatische Genotyp, der westafrikanische Genotyp und der ost-, zentral- und südafrikanische Genotyp. Das Genom des Chikungunya-Virus ist ein positivsträngiges RNA-Genom mit etwa 12000 Nukleotiden. Es enthält zwei offene Leserahmen, wobei der zweite Leserahmen für die Strukturproteine C, E1, E2 und E3 kodiert. Eine vollständige Genomsequenz des Chikungunya-Virus mit der Bezeichnung GD05/2010 ist in der Genbank unter der Zugangsnummer JX088705 erhältlich (Li et al., Journal of Virology (2012), Vol. 86, No. 16, S. 8904-8905).

### d) NS1-Protein des Zika-Virus

Ein weiteres, in dem Test eingesetztes Antigen ist das "non-structural protein 1" (NS1) des Zika-Virus oder ein immunologisch reaktives Teil hiervon mit wenigstens 40 Aminosäuren. Das Genom des Zika-Virus besteht aus einer ca. 10000 Nukleotiden langen RNA, die ein Polyprotein kodiert, das alle strukturellen Proteine aufweist. Die Genomorganisation umfasst die Proteine E, NS1, NS2a, NS2b, NS3, NS4a, NS4b und NS5.

### e) "envelope protein" des Zika-Virus

Das "envelope protein" wird abgekürzt mit "E". Eine komplette Nukleotidsequenz eines Zika-Virus wurde in der Genbank unter der Zugangsnummer KU321639 hinterlegt (Cunha et al., Genome Announcements, März/April 2016, Volume 4, Issue 2, S. 1-2).

In dem erfindungsgemäßen Verfahren werden also sowohl das non-structural protein 1, wie auch das envelope protein eines Zika-Virus eingesetzt.

In einer weiteren Ausführungsform wird auch als Antigen eingesetzt:

### f) NS1-Protein des Gelbfieber-Virus (YFV)

Das Genom des Gelbfieber-Virus kodiert neben dem envelope protein für die NS-Proteine 1-5. Erfindungsgemäß eingesetzt wird ein Peptid aus dem NS1-Protein oder ein Proteinfragment hiervon. Für Reisen in Gelbfieberendemie-Gebiete ist eine Impfung vorgeschrieben, die mit einem attenuierten Virusstamm durchgeführt wird. Es kann daher vorteilhaft sein, bei dem Verfahren auch das NS1-Protein des Gelbfiebervirus einzusetzen.

Erfindungsgemäß wurden einige bevorzugte Konstrukte von Fragmenten bzw. von immunologisch hochrelevanten Peptidfragmenten hergestellt, die bei dem Testverfahren und den Testkits besonders bevorzugt sind. Es handelt sich hierbei um folgende Konstrukte (Teilfragmente) des Envelope Proteins von jeweils Dengue- sowie Zika-Virus. Die getesteten Konstrukte weisen neben den Envelope Protein Sequenzen noch jeweils einen C-terminalen His-Tag und eine Protease-Schnittstelle auf (die Antigensequenzen von DENV und ZIKV sind unterstrichen):
- Domäne III des Envelope Proteins von Dengue-Virus Serotyp 2 (Stamm 16681) mit Protease-Schnittstelle und His-Tag: 
MYSMCTGKFKVVKEIAETQHGTIVIRVQYEGDGSPCKIPFEIMDLEKRHVLGRLITVNPIV
TEKDSPVNIEAEPPFGDSYIIIGVEPGQLKLNWFKKGLEVLFQGPHHHHHHHH
(SEQ ID NO:1).
- Domäne III des Envelope Proteins von Zika-Virus (Stamm SPH2015) mit Protease-Schnittstelle und His-Tag:

MYSLCTAAFTFTKIPAETLHGTVTVEVQYAGTDGPCKVPAQMAVDMQTLTPVGRLITAN
PVITESTENSKMMLELDPPFGDSYIVIGVGEKKITHHWHRSGLEVLFQGPHHHHHHHH
(SEQ ID NO:2).

Diese Fragmente könnten in den Tests bevorzugt verwendet werden.

Bevorzugt eingesetzte Teilfragmente sind folgende NS-1-Peptide, die zur Differenzierung eingesetzt werden können:
- Peptide des NS-1 Proteins. Nachfolgend sind hier nur die Sequenzen für Dengue-Virus Serotyp 2 Stamm 16681; NS-1 aufgeführt; entsprechende Sequenzen können ohne Schwierigkeiten in den Sequenzen von anderen Serotypen und Viren der Familie Flaviviridae aufgefunden werden:
   - KRSLRPQPTELKYSWKTWGKAKMLSTESHNQT (SEQ ID NO:3)
   - PWHLGKLEMDFDFCD (SEQ ID NO:4)
   - DSGCWSWKNKELKCGSGIFITDN (SEQ ID NO:5).

Die Antigene werden auf einer Festphase aufgebracht. Es gibt verschiedene Möglichkeiten, wie dies bewerkstelligt werden kann. In einer bevorzugten Ausführungsform werden die Antigene auf einem Trägerstreifen als Banden aufgebracht. Man spricht in diesem Fall von einem sogenannten Line-Assay. Alternativ hierzu können die Antigene, jeweils räumlich getrennt voneinander, auf ELISA-Testplatten aufgebracht werden, wobei sowohl ein Antigen in einem Reaktionsgefäß wie auch mehrere räumlich voneinander getrennt in einem Reaktionsgefäß eingebracht (Protein-Chip/array) und an die feste Phase gekoppelt werden können. Eine weitere Ausführungsform besteht darin, dass die Antigene an vorzugsweise magnetisierbare Kugeln gekoppelt werden. Dadurch können die Antigene bequem mit der Probe in Kontakt gebracht werden und wieder aus der Reaktionsmischung entfernt werden.

Nach der Reaktion der auf die feste Phase gekoppelten Antigene und der Probe bildet sich ein Immunkomplex aus dem Antigen und den spezifisch hieran bindenden Antikörpern. Um unspezifische Bindungen zu vermeiden, werden einerseits bei der Herstellung der Testkits all diejenigen Stellen abgesättigt, die eine unspezifische Bindung hervorrufen könnten. Es kann sich bei derartigen unspezifischen Bindungsstellen um Teile des Trägermaterials handeln. Nach der Reaktion müssen jedoch Antikörper, die nicht spezifisch gebunden wurden, abgetrennt werden. Dies erfolgt in bevorzugter Ausführungsform durch Waschen mit geeigneten Waschlösungen bzw. Puffern.

Auf der festen Phase hat sich durch die Reaktion des Antigens mit spezifischen, hiergegen gerichteten Antikörpern ein Immunkomplex gebildet, der in ein auswertbares Signal umgewandelt werden muss, sofern in der Probe spezifische Antikörper vorhanden sind.

Für die Umwandlung des auf der Festphase gebildeten Immunkomplexes in ein auswertbares Signal gibt es verschiedene Möglichkeiten. Bevorzugt muss eine derartige Umwandlung in (semi) quantitativer Art und Weise erfolgen, weil es für die Auswertung des Testergebnisses erforderlich ist, dass man die relativen Stärken der Signale miteinander vergleichen kann.

In einer bevorzugten Ausführungsform wird der Immunkomplex auf der Festphase dadurch sichtbar gemacht, dass ein Anti-Antikörper zugegeben wird, der gekoppelt ist mit einem signalgebenden Mittel. Bei den Anti-Antikörpern handelt es sich üblicherweise um in Labortieren (beispielsweise Kaninchen, Ziege) erzeugte polyklonale Antikörper, die entweder gerichtet sind gegen humane IgG-Antikörper oder humane IgM-Antikörper. Die Anti-Antikörper sind üblicherweise gerichtet gegen den Fc-Teil der Antikörper aus der Patientenprobe, die an die jeweiligen Antigene gebunden haben.

Die Anti-Antikörper sind gekoppelt mit einem Signal erzeugenden Mittel. Hierbei kann es sich um ein Mittel handeln, das direkt ein Signal erzeugt, also beispielsweise einen Farbstoff oder einen Fluoreszenz-Farbstoff. Es kann sich aber auch um ein Mittel handeln, das das Signal verstärkt. In bevorzugter Ausführungsform handelt es sich hierbei um ein Enzym, wie beispielsweise Meerrettich-Peroxidase oder alkalische Phosphatase. Das Enzym wandelt ein Substrat in ein messbares Signal, beispielsweise ein gefärbtes Produkt, um. Je länger das Enzym auf das Substrat einwirken gelassen wird, desto stärker ist das gefärbte Signal.

Die Reaktion wird nach einer vorbestimmten Zeit dadurch beendet, dass beispielsweise gewaschen wird.

In dem erfindungsgemäßen Verfahren wird zur Abgrenzung des Rauschens, das immer bei immunologischen Tests auftreten kann, ein sogenannter "Cutoff' eingesetzt. Dieser Cutoff befindet sich beispielsweise auf dem Teststreifen und wird dadurch unter gleichen Bedingungen wie die zu überprüfenden Proben getestet. Der Cutoff des Tests bzw. die Intensität der Cutoff Bande wird so eingestellt, dass definierte negative Probenkollektive unterhalb des Cutoffs liegen und definierte positive Kollektive oberhalb des Cutoffs liegen.

Nach Durchführung des Tests und Umwandlung des Immunkomplexes in ein messbares Signal wird die Intensität des Signals bewertet. Hierbei wird die in Tabelle 1 wiedergegebene Beurteilung zugrundegelegt:

**Tabelle 1: Bewertungsschema**

| **Intensität des Signals** | **Bewertung** |
|---|---|
| keine Reaktion | - |
| Reaktivität unterhalb der Cutoff-Bande | +/- |
| Reaktivität gleich Cutoff-Bande | + |
| Reaktivität stärker als Cutoff-Bande | ++ |
| Reaktivität deutlich stärker als Cutoff-Bande | +++ |
| maximale Reaktivität | ++++ |

Die in Tabelle 1 wiedergegebene Bewertung stellt die visuelle Bewertung dar. Die Messung kann natürlich auch mit geeigneten Geräten, beispielsweise entsprechend eingestellten Photometern, durchgeführt werden. Dann kann die Intensität des Signals einem bestimmten, objektiv messbaren Wert zugeordnet werden.

In bevorzugter Ausführungsform wird das Testverfahren mit derselben Probe für den IgG- und IgM-Nachweis durchgeführt und es wird sowohl das Vorhandensein bzw. die Abwesenheit von IgM und IgG Antikörpern gegen die Antigene bestimmt. Ein hierfür geeigneter Testkit enthält alle hierfür benötigten Reagenzien.

Das erfindungsgemäße Verfahren kann bevorzugt mit entsprechend angepassten Testkits durchgeführt werden. Diese Testkits beinhalten die oben aufgeführten Teststreifen mit den entsprechenden Antigenen, Kontrollbanden (u.a. Cutoff-Bande) und die Reagenzien zum Waschen der Teststreifen oder Test-Mikrotiterplatten. Außerdem enthalten die Testkits häufig Positiv- und Negativ-Kontrollseren, sowie Reaktionskontrollen.

Die Erfindung wird in den Beispielen weiter erläutert:

### Beispiel 1

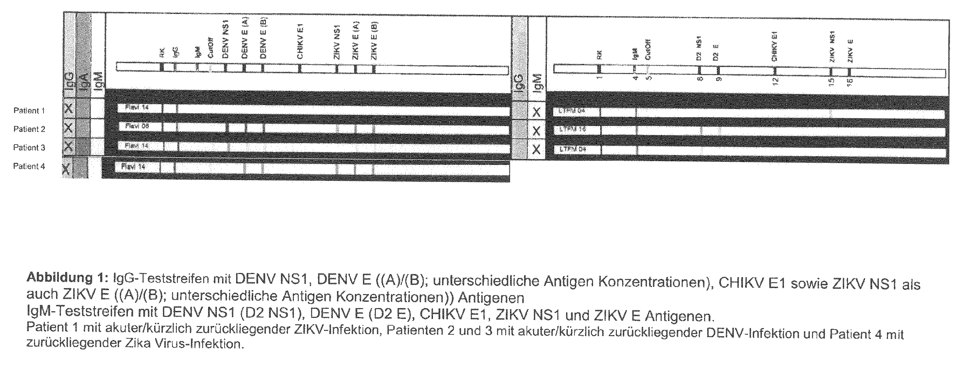

Es wurde ein sogenannter "Line-Assay"-Teststreifen mit den erfindungsgemäß eingesetzten Antigenen hergestellt, wobei die einzelnen Antigene an unterschiedlichen Stellen des Teststreifens aufgebracht wurden. Diese Teststreifen wurden mit Seren von vier verschiedenen Patienten umgesetzt. Die Ergebnisse sind in Abbildung 1 dargestellt. Hierbei handelte es sich um:
Patient 1: Der Patient zeigte keinerlei IgG Antikörperreaktionen gegen Dengue-, Chikungunya- und Zika-Virus, aber IgM Antikörper gegen das ZIKV NS1 Protein. Damit hat der Patient eine frische oder kürzlich zurückliegende Zika Virus-Infektion. Die Infektion ist relativ einfach nachzuweisen, da es sich um eine singuläre, Flavivirus-Erstinfektion handelt.

Patient 2: Der Patient hat sowohl IgG Antikörper gegen Dengue- als auch gegen Zika-Virus. Im direkten Vergleich der korrespondierenden Antikörper gegen DENV/ZIKV NS1 stellt sich dar, dass der Antikörpertiter von Dengue-Virus NS1 deutlich stärker ist (++++) als der korrespondierende von Zika-Virus NS1 (++). Das gleiche gilt für die korrespondierenden E Proteine von Dengue- (+++) und Zika-Virus (++). Im direkten Vergleich der IgG Antikörpertiter ist es daher möglich, eine Unterscheidung zwischen anti-Dengue- und anti-Zika Virus IgG-Antikörpern vorzunehmen. Desweiteren weist der Patient IgM-Antikörper, die ausschließlich gegen die Dengue-Virus Antigene gerichtet sind, auf. Anhand der IgM-Antwort (Dengue-Virus NS1 (++), Dengue-Virus E (++)) läßt sich die Aussage treffen, dass der Patient eine akute bzw. kürzlich zurück liegende DENV-Infektion hatte.

Patient 3: Der Patient hat sowohl IgG Antikörper gegen Dengue- als auch Antikörper gegen Zika-Virus. Im direkten Vergleich der korrespondierenden Antikörper gegen DENV/ZIKV NS1 stellt sich dar, dass der Antikörpertiter von Dengue-Virus NS1 deutlich höher (stärker) ist (++) als der korrespondierende von Zika-Virus NS1 (+/-). Anhand der korrespondierenden E Proteine von Dengue-Virus (+) und Zika-Virus (+) kann nicht differenziert werden. Auch hier ist es möglich, im Vergleich der anti-NS-1 IgG Antikörpertiter eine Aussage zu treffen. Weiterhin weist der Patient IgM Antigkörper gegen das NS-1 Protein von Dengue-Virus (++) auf. Der Patient hat eine akute bzw. kürzlich zurück liegende DENV-Infektion.

Patient 4: Der Patient hat keine IgG Antikörper gegen Dengue-Virus NS-1 (-), aber Antikörper gegen Zika-Virus NS-1 (++). Die Antikörper gegen DENV E (++) als auch ZIKV E lassen keine Differenzierung zu. Durch die gegen NS-1 gerichteten Antikörper ist es hier möglich, auf eine Zika-Virus Infektion zu schließen. Der IgM-Teststreifen, hier nicht dargestellt, ist allerdings negativ. Dies lässt vermuten, dass es sich um eine zurückliegende Infektion handelt.

In der Regel ist die IgM-Immunantwort gegen Flaviviren spezifischer im Vergleich zur IgG-Immunantwort. Dies gilt insbesondere für eine Flaviviren-Sekundärinfektion. Da die IgM-Antwort bei einer Sekundärinfektion jedoch i.d.R. deutlich niedriger ausfällt oder gar nicht nachweisbar ist, ist es von Vorteil, wenn mittels IgG-Immunantwort ein Hinweis auf den Krankheitserreger erhalten werden kann. Über den direkten Vergleich der Antikörperantworten gegen z.B. NS-1 im IgG-Test ergeben sich oftmals Hinweise auf den Infektionserreger. Hierbei ist aber zu beachten, dass bei frischen Infektionen (Auftreten der Symptome ≤ 4 Tagen) gegen den aktuellen Erreger die IgG-Antwort i.d.R. noch nicht erfolgt ist. Das heißt, bei Proben aus der Akutphase ist hier eine Folgeprobe im Abstand von bevorzugt 1-2 Wochen notwendig.

Zusammenfassend wurde die Auswertung des Tests in der nachfolgenden Tabelle 2 dargestellt:
Auswertung: die verschiedenen Intensitäten der Banden sind mit -, +/-, +, ++, +++, ++++ dargestellt. RK ist eine Reaktionskontrolle, die erscheinen muss. IgG oder IgM gibt die jeweilige Immunglobulin Klasse an. Die Banden werden immer im Vergleich zur Cutoff Bande ausgewertet. DENV NS1, DENV E (A), DENV E (B), CHIKV E1, ZIKV NS1, ZIKV E (A) und ZIKV E (B). Variante (A) und (B) entsprechen jeweils zwei unterschiedlichen Antigenkonzentrationen.

**Tabelle 2: Zusammenfassung Auswertung Patienten 1-4**

| Patient | IgG/M | DENV NS1 / D2 NS1 | DENV E (A) / D2 E | DENV E (B) / n.a. | CHIKV E1 / CHIKV E1 | ZIKV NS1 / ZIKV NS1 | ZIKV E (A) / ZIKV E | ZIKV E (B) / n.a. |
|---|---|---|---|---|---|---|---|---|
| 1 | IgG | - | - | - | - | - | - | - |
| 1 | IgM | - | - | n.a. | - | ++ | - | n.a. |
| 2 | IgG | ++++ | +++ | +++ | - | ++ | ++ | ++ |
| 2 | IgM | ++ | ++ | n.a. | - | - | - | n.a. |
| 3 | IgG | ++ | + | + | - | +/- | + | + |
| 3 | IgM | ++ | - | n.a. | - | - | - | n.a. |
| 4 | IgG | - | ++ | ++ | - | ++ | ++ | ++ |
| 4 | IgM | n.a. | | | | | | |

### Beispiel 2

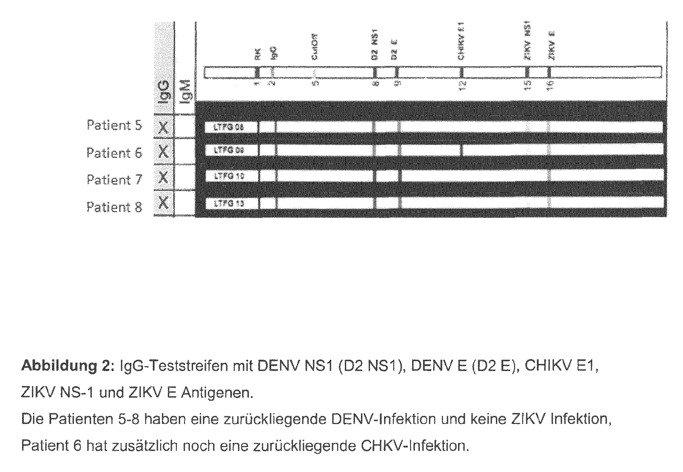

Es wurde weiterhin ein Line-Assay mit Seren von Patienten durchgeführt, bei denen eine Differenzierung zwischen Dengue-Virus und Zika-Virus durch einen Vergleich der Intensität der Banden möglich ist. Die Ergebnisse sind in Abbildung 2 dargestellt.

Die Abbildung 2 enthält Beispielstreifen mit Proben von Patienten 5-8, bei denen eine Differenzierung zwischen DENV und ZIKV durch direkten Vergleich der DENV und ZIKV NS-1-Bandenintensität möglich ist. Die IgM-Testergebnisse sind negativ, was einen Hinweis auf zurückliegende Infektionen gibt.

Sowohl DENV als auch ZIKV NS-1 sind positiv (reagieren stärker im Vergleich zur Cutoff-Bande). Aber anhand des direkten Intensitätsvergleichs beider Antigene kann man erkennen, dass auf den Teststreifen die jeweilige DENV NS-1-Bande deutlich stärker gefärbt ist im Vergleich zur ZIKV NS-1-Bande.

Die Patienten 5-8 haben eine zurückliegende DENV-Infektion und keine ZIKV Infektion, Patient 6 hat zusätzlich noch eine zurückliegende CHKV-Infektion.

### Beispiel 3

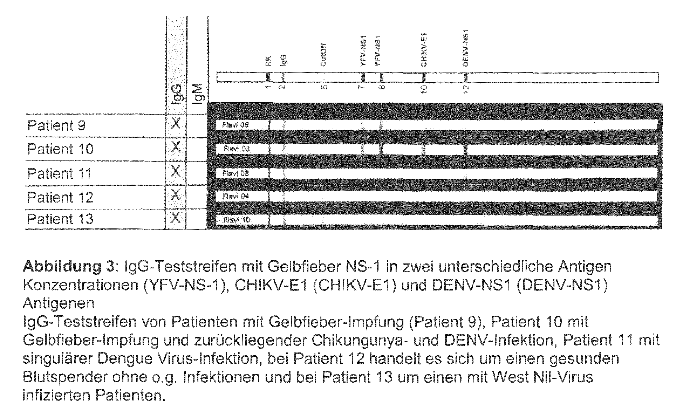

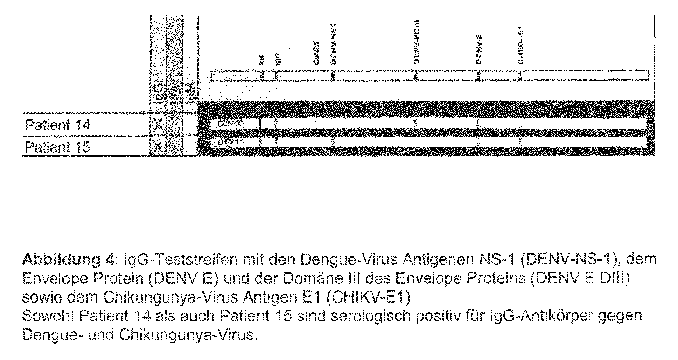

Die Abbildung 3 enthält Beispielstreifen mit Proben von Patienten 9-13, bei denen eine Differenzierung zwischen Gelbfieber-, Chikungunya-, Dengue- und West Nil- Virus möglich ist.

Patient 9 wurde innerhalb der letzten zwei Jahre gegen Gelbfieber geimpft und zeigt entsprechend im IgG-Nachweis eine Reaktion mit dem Gelbfieber-Virus NS-1 Antigen, es liegt keinerlei Kreuzreaktivität zu anderen Erregern vor. Patient 10 ist gegen Gelbfieber geimpft und weist desweiteren IgG-Antikörper gegen Chikungunya- und Dengue-Virus auf. Es reagieren die NS-1 Antigene des Gelbfieber- und des Dengue-Virus sowie das E1 Antigen des Chikungunya-Virus. Patient 11 ist ein Beispiel für eine zurückliegende singuläre Dengue-Virus Infektion (Patient mit Dengue-Fieber vor 7 Jahren) und man sieht, dass keinerlei Kreuzreaktivität zu Gelbfieber- (Flavivirus) und Chinkungunya-Virus vorliegt. Bei Patient 12 handelt es sich um einen gesunden Blutspender aus Deutschland ohne o.g. Infektionen, welcher als Negativkontrolle fungiert.

Bei Patient 13 handelt es sich um einen mit West Nil Virus infizierten Patienten, er zeigt keinerlei Kreuzreaktivität zu den anderen Erregern.

### Beispiel 4

Es wurden zwei Teststreifen hergestellt, die einerseits das Dengue-Virus (Serotyp 2) Envelope Protein (DENV E) sowie ein Teilfragment des Envelope Proteins bestehend aus der Domäne III (DENV EDIII) enthielten. Die Teststreifen wurden umgesetzt mit Serum der Patienten 14 und 15, wobei beide Patienten auch serologisch positiv sind für IgG-Antikörper gegen Dengue- und Chikungunya-Virus. Das Beispiel belegt, dass der Test nicht nur mit den Volllängen-Antigenen, sondern auch mit geeigneten Fragmenten durchgeführt werden kann.

## Patentansprüche

1. Verfahren zur immunologischen Diagnose einer Probe eines Patienten mit einer potentiellen Infektion eines Arbovirus, worin
a) eine Probe mit mehreren voneinander getrennt auf einer Festphase aufgebrachten Antigenen in Kontakt gebracht wird, wobei wenigstens folgende Antigene eingesetzt werden:
aa) das "non-structural protein 1" eines ersten Arbovirus oder ein immunologisch reaktives Teil hiervon mit wenigstens 8 Aminosäuren,
bb) ein "envelope protein" eines ersten Arborvirus oder ein immunologisch reaktives Teil hiervon mit wenigstens 8 Aminosäuren,
cc) ein "envelope protein " eines zweiten Arbovirus oder ein immunologisch reaktives Teil hiervon mit wenigstens 8 Aminosäuren,
dd) das "non-structural protein 1" eines dritten Arbovirus oder ein immunologisch reaktives Teil hiervon mit wenigstens 8 Aminosäuren und
ee) ein "envelope protein" eines dritten Arbovirus oder ein immunologisch reaktives Teil hiervon mit wenigstens 8 Aminosäuren;
b) Waschen der Festphase, um unspezifische Bindungen abzutrennen,
c) Umwandeln des auf der Festphase gebildeten Immunkomplexes in ein Signal und
d) Auswerten des Testverfahrens durch Vergleich der relativen Signalstärken.

2. Verfahren nach Anspruch 1, **dadurch gekennzeichnet, dass** in dem Diagnoseverfahren weiterhin das folgende Antigen eingesetzt wird:
ff) ein Peptid mit wenigstens 8 Aminosäuren aus der NS1-Region eines vierten Arbovirus.

3. Verfahren nach einem der Ansprüche 1 oder 2, **dadurch gekennzeichnet, dass** das erste Arbovirus ein Dengue-Virus Serotyp 2, das zweite Arbovirus ein Chikungunya-Virus, das dritte Arbovirus ein Zika-Virus und das vierte Arbovirus ein Gelbfieber-Virus ist.

4. Verfahren nach einem der Ansprüche 1 bis 3, **dadurch gekennzeichnet, dass** die Umwandlung des auf der Festphase gebildeten Immunkomplexes in ein Signal durch Anti-Antikörper erfolgt, die gekoppelt sind mit einem Signal-erzeugenden Mittel.

5. Verfahren nach Anspruch 4, **dadurch gekennzeichnet, dass** die Anti-Antikörper humane Anti-IgG-Antikörper und/oder humane Anti-IgM-Antikörper sind.

6. Verfahren nach einem der Ansprüche 4 oder 5, **dadurch gekennzeichnet, dass** das farbgebende Mittel ein Enzym ist, das ein Substrat in ein gefärbtes Produkt umwandelt, das visuell sichtbar ist und/oder mit einer geeigneten Vorrichtung quantitativ messbar ist.

7. Verfahren nach einem der Ansprüche 1 bis 6, **dadurch gekennzeichnet, dass** für jede Probe bestimmt wird, ob die Antikörper zu der Klasse der IgM, der IgG oder zu beiden gehören.

8. Verfahren nach einem der vorhergehenden Ansprüche, **dadurch gekennzeichnet, dass** die Auswertung der Teststreifen durch einen Vergleich der Intensitäten der Signale der einzelnen Antigene erfolgt, wobei die einzelnen Antigene auf einen "Cutoff" referenziert werden und wobei für jedes Antigen die Intensität des Signals bestimmt wird, wobei folgende Beurteilung zugrundegelegt wird:
| **Intensität des Signals** | **Bewertung** |
|---|---|
| keine Reaktion | - |
| Reaktivität unterhalb der Cutoff-Bande | +/- |
| Reaktivität gleich Cutoff-Bande | + |
| Reaktivität stärker als Cutoff-Bande | ++ |
| Reaktivität deutlich stärker als Cutoff-Bande | +++ |
| maximale Reaktivität | ++++ |

9. Verfahren nach einem der vorhergehenden Ansprüche, **dadurch gekennzeichnet, dass** es sich um einen ELISA-Test handelt, wobei in jedes Reaktionsgefäß ein Antigen eingebracht wird, oder dass es sich um einen Line-Assay handelt.

10. Testkit zur Durchführung eines Verfahrens nach einem der Ansprüche 1 bis 9, **dadurch gekennzeichnet, dass** das Testkit mehrere voneinander räumlich getrennte Festphasen beinhaltet, wobei das Testkit wenigstens folgende Antigene aufweist:
aa) das "non-structural protein 1" eines ersten Arbovirus oder ein immunologisch reaktives Teil hiervon mit wenigstens 8 Aminosäuren,
bb) ein "envelope protein" eines ersten Arbovirus oder ein immunologisch reaktives Teil hiervon mit wenigstens 8 Aminosäuren,
cc) ein "envelope protein" eines zweiten Arborvirus oder ein immunologisch reaktives Teil hiervon mit wenigstens 8 Aminosäuren,
dd) das "non-structural protein 1" eines dritten Arbovirus oder ein immunologisch reaktives Teil hiervon mit wenigstens 8 Aminosäuren und
ee) ein "envelope protein" eines dritten Arbovirus oder ein immunologisch reaktives Teil hiervon mit wenigstens 8 Aminosäuren
sowie Reagenzien zum Waschen des Immunkomplexes und Reagenzien zur Umwandlung des Immunkomplexes in ein optisch sichtbares Signal und ein voreingestelltes Serum, das als Cutoff-Probe eingesetzt wird.

11. Testkit nach Anspruch 11, **dadurch gekennzeichnet, dass** das Testkit weiterhin das folgende Antigen aufweist:
ff) ein Peptid mit wenigstens 8 Aminosäuren aus der NS1-Region eines vierten Arbovirus.

12. Testkit nach Anspruch 10 oder 11, **dadurch gekennzeichnet, dass** das erste Arbovirus ein Dengue-Virus Serotyp 2, das zweite Arbovirus ein Chikungunya-Virus, das dritte Arbovirus ein Zika-Virus und das vierte Arbovirus ein Gelbfieber-Virus ist.

13. Verfahren nach einem der Ansprüche 1 bis 9 sowie Testkit nach einem der Ansprüche 10-12, **dadurch gekennzeichnet, dass** der immunologisch reaktive Teil der Antigene wenigstens 40 Aminosäuren aufweist.

14. Verfahren nach einem der Ansprüche 1 bis 9 sowie Testkit nach einem der Ansprüche 10-12, **dadurch gekennzeichnet, dass** der immunologisch reaktive Teil der eingesetzten Antigene wenigstens 80 % der vollen Länge des natürlich auftretenden Antigens umfasst.

15. Verfahren nach einem der Ansprüche 1 bis 9 sowie Testkit nach einem der Ansprüche 10-12, **dadurch gekennzeichnet, dass** der reaktive Teil der eingesetzten Antigene wenigstens 95 % der vollen Länge des natürlich auftretenden Antigens umfasst.


SEQUENZPROTOKOLL

<110>  Mikrogen GmbH

<120>  Verfahren zur immunologischen Diagnose einer Probe mit einer
       potentiellen Infektion eines Arbovirus und hierfür geeignete
       Testkits

<130>  PEP02824MIK

<160>  5

<170>  PatentIn version 3.5

<210>  1
<211>  114
<212>  PRT
<213>  Dengue virus

<400>  1

Met Tyr Ser Met Cys Thr Gly Lys Phe Lys Val Val Lys Glu Ile Ala
1               5                   10                  15

Glu Thr Gln His Gly Thr Ile Val Ile Arg Val Gln Tyr Glu Gly Asp
            20                  25                  30

Gly Ser Pro Cys Lys Ile Pro Phe Glu Ile Met Asp Leu Glu Lys Arg
        35                  40                  45

His Val Leu Gly Arg Leu Ile Thr Val Asn Pro Ile Val Thr Glu Lys
    50                  55                  60

Asp Ser Pro Val Asn Ile Glu Ala Glu Pro Pro Phe Gly Asp Ser Tyr
65                  70                  75                  80

Ile Ile Ile Gly Val Glu Pro Gly Gln Leu Lys Leu Asn Trp Phe Lys
                85                  90                  95

Lys Gly Leu Glu Val Leu Phe Gln Gly Pro His His His His His His
            100                 105                 110

His His

<210>  2
<211>  117
<212>  PRT
<213>  Zika virus

<400>  2

Met Tyr Ser Leu Cys Thr Ala Ala Phe Thr Phe Thr Lys Ile Pro Ala
1               5                   10                  15

Glu Thr Leu His Gly Thr Val Thr Val Glu Val Gln Tyr Ala Gly Thr
            20                  25                  30

Asp Gly Pro Cys Lys Val Pro Ala Gln Met Ala Val Asp Met Gln Thr
        35                  40                  45

Leu Thr Pro Val Gly Arg Leu Ile Thr Ala Asn Pro Val Ile Thr Glu
    50                  55                  60

Ser Thr Glu Asn Ser Lys Met Met Leu Glu Leu Asp Pro Pro Phe Gly
65                  70                  75                  80

Asp Ser Tyr Ile Val Ile Gly Val Gly Glu Lys Lys Ile Thr His His
                85                  90                  95

Trp His Arg Ser Gly Leu Glu Val Leu Phe Gln Gly Pro His His His
            100                 105                 110

His His His His His
        115

<210>  3
<211>  32
<212>  PRT
<213>  Dengue virus

<400>  3

Lys Arg Ser Leu Arg Pro Gln Pro Thr Glu Leu Lys Tyr Ser Trp Lys
1               5                   10                  15

Thr Trp Gly Lys Ala Lys Met Leu Ser Thr Glu Ser His Asn Gln Thr
            20                  25                  30

<210>  4
<211>  15
<212>  PRT
<213>  Dengue virus

<400>  4

Pro Trp His Leu Gly Lys Leu Glu Met Asp Phe Asp Phe Cys Asp
1               5                   10                  15

<210>  5
<211>  24
<212>  PRT
<213>  Dengue virus

<400>  5

Asp Ser Gly Cys Val Val Ser Trp Lys Asn Lys Glu Leu Lys Cys Gly

1                   5                        10                       15

Ser Gly Ile Phe Ile Thr Asp Asn
            20